(19) Europäisches Patentamt
European Patent Office
Office européen des brevets

(11) Numéro de publication : **0 419 860 B1**

# (12) FASCICULE DE BREVET EUROPEEN

(45) Date de publication du fascicule du brevet : **04.08.93 Bulletin 93/31**

(51) Int. Cl.$^5$ : **C07C 43/196,** C07C 49/175, C07C 47/198, C07C 41/30, A61K 7/00

(21) Numéro de dépôt : **90116301.4**

(22) Date de dépôt : **25.08.90**

## (54) Composés alicycliques oxygénés, leur utilisation à titre d'ingrédients parfumants et procédé pour leur préparation.

(30) Priorité : **25.09.89 CH 3472/89**
**03.10.89 CH 3613/89**

(43) Date de publication de la demande : **03.04.91 Bulletin 91/14**

(45) Mention de la délivrance du brevet : **04.08.93 Bulletin 93/31**

(84) Etats contractants désignés : **CH DE FR GB LI NL**

(56) Documents cités : **EP-A- 0 080 148**

(56) Documents cités :
**EP-A- 0 081 699**
**EP-A- 0 121 828**
**FR-A- 2 418 214**
**US-A- 1 873 430**

(73) Titulaire : **FIRMENICH SA**
**1, route des Jeunes**
**CH-1211 Genève 8 (CH)**

(72) Inventeur : **Chapuis, Christian**
**12, Chemin du Champ d'Anier**
**CH-1209 Petit-Saconnex (CH)**

(74) Mandataire : **Salvadori, Giuseppe, Dr.**
**c/o Firmenich S.A. Case Postale 239**
**CH-1211 Genève 8 (CH)**

EP 0 419 860 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a trait au domaine de la parfumerie. Elle concerne, plus particulièrement, des composés de formule

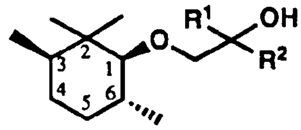

(I)

dans laquelle $R^1$ représente un atome d'hydrogène ou un radical alkyle saturé de $C_1$ à $C_4$, linéaire ou ramifié, et $R^2$ représente un radical alkyle inférieur, saturé ou insaturé, linéaire ou ramifié, ou des mélanges riches en l'un de ces composés.

Par radical alkyle inférieur on entend ici un radical alkyle contenant de 1 à 4 atomes de carbone.

Un mélange riche en composé de formule (I) s'entend ici un mélange contenant une quantité prépondérante dudit composé accompagné de son isomère de formule

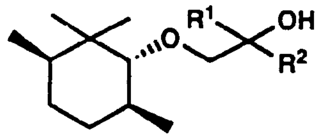

(II)

dans laquelle $R^1$ et $R^2$ ont le sens indiqué à la formule (I).

L'invention concerne également l'utilisation, à titre d'ingrédient parfumant, d'un composé de formule (I) ou d'un mélange tel que défini ci-dessus et un procédé pour la préparation des composés de formule (I).

Le brevet européen n° 0081699 décrit des composés de formule

$$X' - CH_2 - CH(OH) - CH_2 - R'$$

dans laquelle le symbole R' représente un radical alkyle contenant de 1 à 3 atomes de carbone et X' un radical cyclohexyloxy substitué de formule

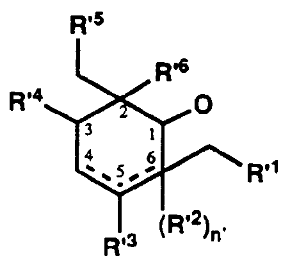

dans laquelle l'indice n' vaut 0 ou 1, les pointillés désignent l'emplacement d'une simple (n'=1) ou double liaison en position 2 (n'=0) ou 3 (n'=1) et chacun des symboles $R'^1$ à $R'^6$ représente un atome d'hydrogène ou un radical méthyle. Il est également décrit dans ce brevet que ces composés possèdent des propriétés odorantes intéressantes et qu'ils se distinguent notamment par leur "odeur boisée-ambrée élégante, chaude et montante, enrichie d'une tonalité légèrement animale rappelant celle de l'ambre naturel, parfois faiblement fruitée ou fleurie selon les cas". Finalement, on mentionne aussi que les composés précités peuvent se présenter sous diverses formes stéréoisomériques, qui peuvent développer, à côté de leur note boisée-ambrée commune, d'autres nuances olfactives. On cite, cependant, que l'on préfère utiliser les mélanges de stéréoisomères pour des raisons d'ordre pratique et économique.

Il ressort de l'art antérieur précité qu'aucun intérêt particulier n'a été reconnu dans l'une ou l'autre de ces formes stéréoisomériques et que leurs propriétés organoleptiques individuelles n'ont pas été reconnues ou jugées suffisamment distinctives pour justifier l'emploi d'un isomère en particulier, plutôt que du mélange d'isomères. C'est ainsi que les exemples d'utilisation de ces composés en parfumerie décrits dans ledit brevet ne concernent que des mélanges d'isomères indiscriminés, alors qu'au moins pour l'un des composés illustrés

deux formes stéréoisomériques ont été décrites, à savoir les composés de formule

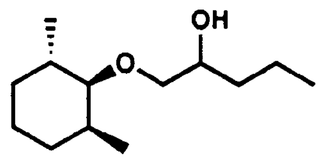

et

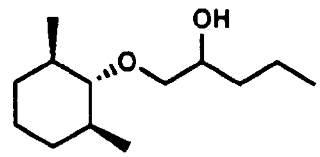

Ce n'est pas non plus la description olfactive de ces deux isomères, à savoir respectivement légèrement boisé et boisé-transpiration, qui aurait permis à l'homme du métier de prévoir une utilité particulière quelconque pour les différentes formes stéréoisomériques de ces composés ou de composés analogues.

C'est donc de façon surprenante que nous avons maintenant découvert que les composés nouveaux de formule (I) et les mélanges selon l'invention possédaient des propriétés odorantes fort intéressantes et pouvaient de ce fait servir à compléter et diversifier la palette du parfumeur dans le domaine des composés à odeur boisée.

Nous avons en effet pu constater qu'il existe une différence marquée entre les qualités olfactives des composés de formule (I) et, en particulier, celles de leurs stéréoisomères cis de formule

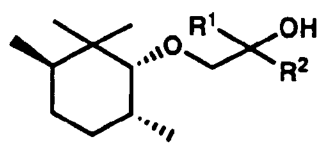

(III)

ou

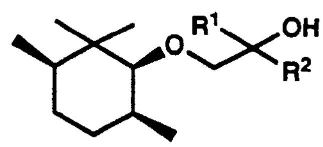

(IV)

dans lesquelles $R^1$ et $R^2$ sont définis comme à la formule (I). Lorsqu'on parle ici de configurations cis et trans, on se réfère en particulier à l'orientation relative des groupes substituants en positions 1 et 6 du cycle.

Les composés de formule (I), et lesdits mélanges riches en ces composés, se révèlent être des ingrédients parfumants nettement supérieurs, de par la puissance, ténacité et caractère de leur note odorante, à leurs isomères cis et, de ce fait, aux mélanges de stéréoisomères contenant des quantités considérables de composés de formule (III) et (IV).

C'est ainsi que lors d'un test d'évaluation comparatif entre un composé préféré selon l'invention, à savoir le 1-(2,2,c-3,t-6-tétraméthyl-r-1-cyclohexyloxy)-2-pentanol, et un mélange contenant 51% en poids de ce composé, 28% de l'isomère de formule (III) correspondant ou 1-(2,2,t-3,c-6-tétraméthyl-r-1-cyclohexyloxy)-2-pentanol et 21% de l'isomère de formule (IV) correspondant ou 1-(2,2,c-3,c-6-tétraméthyl-r-1-cyclohexyloxy)-2-pentanol, on s'est aperçu que les différences olfactives entre ces deux ingrédients parfumants étaient considérables. En effet, le mélange susmentionné possédait une note boisée-ambrée, avec un côté animal dont la netteté était obscurcie par une nuance de transpiration. On pouvait également identifier une nuance presque fruitée et poussiéreuse. Pour sa part, le composé selon l'invention ou 1-(2,2,c-3,t-6-tétraméthyl-r-1-cyclohexyloxy)-2-pentanol, présentait une odeur boisée-ambrée dont la note animale était nette et dominante, sans posséder la direction transpiration. L'odeur était beaucoup plus claire, plus nette et plus puissante. Par ailleurs, l'évaluation des isomères de structure cis a permis d'établir que ce sont eux qui apportent la note fruitée, poussiéreuse et de transpiration identifiée dans le mélange susmentionné et qui réduit la valeur olfactive de ce mélange par rapport à celle du 1-(2,2,c-3,t-6-tétraméthyl-r-1-cyclohexyloxy)-2-pentanol.

Les différences de propriétés odorantes entre le mélange de stéréoisomères susmentionné, obtenu selon l'art antérieur cité, et des composés de formule (I) selon la présente invention ressortent également des exemples d'application de ces ingrédients parfumants dans des compositions et bases parfumantes, présentés plus loin.

Les propriétés odorantes des composés de formule (I) apparaissent d'autant plus surprenantes que l'on sait que l'art antérieur fait état de maints exemples d'utilisation de composés odorants boisés de structure similaire à celle des composés selon l'invention, et que l'on aurait pu s'attendre à ce que ces derniers n'apportent rien d'original et inattendu à la palette du parfumeur.

Dans ce contexte, il y a lieu de citer le brevet européen n° 0121828 qui a trait à l'utilisation en parfumerie des alcools cycloaliphatiques de formule

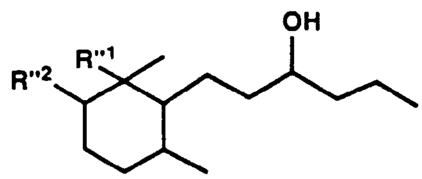

(V)

dans laquelle chacun des symboles R"[1] et R"[2] sert à désigner un radical méthyle, ou R"[1] désigne un radical éthyle et R"[2] un radical méthyle ou l'hydrogène. Les formes stéréoisomériques trans de ces composés, définies à l'aide de la formule

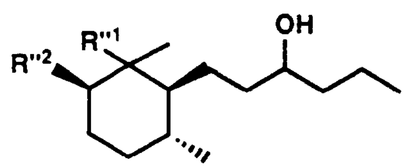

sont jugées nettement supérieures à leurs isomères cis, du point de vue olfactif, et possèdent des notes odorantes du type boisé-ambré, avec un caractère animal très prononcé. Il s'agit de notes odorantes décrites comme étant d'une rare puissance. Parmi ces composés, le 1-(2,2,3,6-tétraméthyl-1-cyclohexyl)-3-hexanol [mélange constitué pour l'essentiel de l'isomère c-3,t-6] est un ingrédient parfumant fort apprécié pour sa note animale très puissante et il sera cité ici comme terme de comparaison vis-à-vis des composés selon la présente invention.

Le tableau (I) présenté ci-après résume les propriétés odorantes de composés préférés selon la présente invention et compare la puissance, ténacité et valeur olfactive de leur note avec celles de la note du 1-(2,2,3,6-tétraméthyl-1-cyclohexyl)-3-hexanol susmentionné. Il ressort de ce tableau qu'en dépit de la ressemblance structurelle des composés de formule (I) cités, chacun de ces composés présente des propriétés odorantes distinctes de celles des autres composés analogues. Ainsi, chacun de ces composés peut trouver un emploi particulier suivant l'effet parfumant recherché et la nature du produit que l'on désire parfumer. Par ailleurs, il ressort également de ce tableau que, malgré la moindre puissance et ténacité des notes odorantes de ces composés, par rapport à celles de la note du composé précité connu de l'art antérieur, la performance des composés selon l'invention, et notamment celle des 1-(2,2,c-3,t-6-tétraméthyl-r-1-cyclohexyloxy)-2-pentanol, 1-(2,2,c-3,t-6-tétraméthyl-r-1-cyclohexyloxy)-4-pentén-2-ol ou 1-(2,2,c-3,t-6-tétraméthyl-r-1-cyclohexyloxy)-2-butanol, cités à titre plus préférentiel, se révèle être tout aussi bonne de par la qualité de leurs notes olfactives. Leur utilisation dans des compositions parfumantes peut même être plus "facile" que celle dudit composé connu, du fait que leur note animale est moins agressive et dominante que celle de ce dernier. Ainsi, les composés (I) peuvent constituer, suivant le type d'application, des ingrédients parfumants d'un emploi alternatif à celui du 1-(2,2,3,6-tétraméthyl-1-cyclohexyl)-3-hexanol et ils forment entre eux un ensemble olfactif nuancé qui enrichit la palette du parfumeur, chacun de ces composés pouvant trouver un emploi privilégié dans une composition particulière. Ceci ressort clairement des exemples d'application présentés plus loin.

TABLEAU I - Propriétés odorantes des composés de formule (I) et évaluation comparative avec celles du 1-(2,2,c-3,t-6-tétraméthyl-r-1-cyclohexyl)-3-hexanol

| Référence | Composé | Evaluation olfactive |
|---|---|---|
| A) | 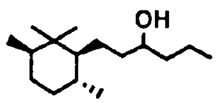 | note boisée, ambrée, animale très puissante |
| 1) | 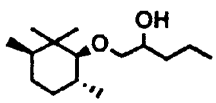 | note bien boisée-ambrée, chaude, élégante et profonde ; plus veloutée que celle de A), moins tenace et moins puissante mais toujours excellente ; d'emploi plus facile que A) pour le même type d'applications |
| 2) | 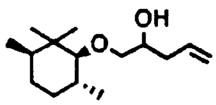 | note similaire à celle de 1), aussi riche et boisée mais allégée par une tonalité fruitée, fleurie, crémeuse et vaguement épicée ; moins tenace que 1), puissance comparable |
| 3) | 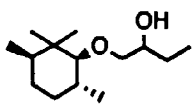 | caractère bien boisé, note plus tenace que celle de 2), comparable à celle de 1), mais avec une nuance ironée et fleurie, rappelant les salicylates |
| 4) | 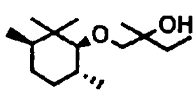 | note boisée-ambrée avec un côté "crayon", poussière, moins puissante que les précédents, note finale épicée, faible ténacité |
| 5) | 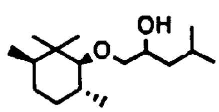 | note moins ambrée et plus faible que celle de 1), nuance "chaux", papier mâché, poussière ; bonne ténacité comparable à celle de 3) |
| 6) | 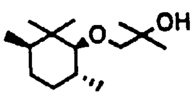 | note boisée, nuance poussière, vieille planche de bois de chantier, moins de caractère ambré ; bonne ténacité comparable à 3) |

TABLEAU I (Suite)

| Référence | Composé | Evaluation olfactive |
|---|---|---|
| 7) | 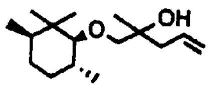 | note boisée-ambrée, de puissance moyenne, nuances terreuses, moisies |
| 8) | 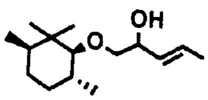 | note boisée, camphrée, presque bornéolée, ténacité moyenne plus faible que les précédents |

Comme cité précédemment, les mélanges d'isomères contenant une quantité prépondérante de composé (I), accompagné de son isomère de configuration trans de formule (II), font également l'objet de la présente invention. Par quantité prépondérante on entend ici toute proportion de composé (I) égale ou supérieure à 60% en poids, par rapport au poids du mélange. Selon l'invention, les mélanges d'isomères contenant 75% ou plus en poids de composé (I), accompagné d'une quantité inférieure ou égale à 25% en poids de composé de formule (II) sont préférées.

D'autre part, bien que les isomères de configuration cis représentés par la formule (III) ou (IV) définie précédemment soient nettement inférieures, du point de vue olfactif, aux composés correspondants de formule (I) ou (II), il a été constaté que les mélanges d'isomères ne contenant pas plus de 10% en poids, globalement, de composé (III) et/ou (IV) possédaient des propriétés olfactives qui convenaient parfaitement au but de la présente invention et qui pouvaient s'avérer avantageuses pour certaines applications moins onéreuses.

Il est clair que les nouveaux mélanges d'isomères selon l'invention sont nettement avantageux par rapport aux mélanges d'isomères connus de l'état de la technique cité, lesquels contenaient des quantités beaucoup plus élevées de composés de configuration cis.

Les composés de formule (I) ou les mélanges selon l'invention peuvent être utilisés aussi bien en parfumerie fine qu'en parfumerie fonctionnelle. Ils conviennent particulièrement bien à la préparation de compositions parfumantes fort variées, notamment de type fleuri, auxquelles ils apportent un fond boisé, riche et élégant, sans que la tonalité animale qui l'accompagne ne devienne trop dominante. Ils trouvent également un emploi apprécié dans la préparation de produits parfumés variés, tels que savons, gels de douche ou bain, shampoings, préparations cosmétiques, désodorisants corporels ou d'air ambiant, détergents, adoucissants textiles, ou encore dans des parfums et eaux de toilette, ainsi que dans des lotions après rasage.

Les composés de formule (I) peuvent être utilisés aux fins décrites ci-dessus dans des proportions variant dans une gamme de valeurs étendue. L'homme du métier sait par expérience que ces proportions dépendent de l'effet parfumant désiré, ainsi que de la nature du produit que l'on désire parfumer. C'est ainsi que des concentrations de l'ordre de 1 à 10%, voire même 20%, en poids, par rapport au poids de composition dans laquelle ils sont incorporés, peuvent être utilisées. Ces concentrations peuvent être inférieures aux valeurs citées lors du parfumage d'articles divers tels les savons, les articles de toilette, les cosmétiques ou les détergents ou adoucissants textiles.

De par leur propriétés olfactives particulières, les composés (I) peuvent trouver un domaine d'application fort étendu, soit par addition directe aux produits que l'on désire parfumer soit, plus fréquemment, par dilution préalable dans un solvant usuel, comme l'éthanol, l'anozol ou le phtalate diéthylique et en mélange avec d'autres ingrédients parfumants naturels ou synthétiques de nature variée.

Les composés de formule (I) et les mélanges riches en ces composés sont préparés selon un procédé original qui fait également l'objet de la présente invention. Ce procédé est caractérisé en ce qu'on fait réagir, dans les conditions d'une réaction de Grignard, un composé de formule

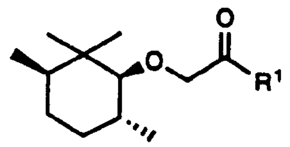

(VI)

dans laquelle $R^1$ représente un atome d'hydrogène ou un radical alkyle saturé de $C_1$ à $C_4$, linéaire ou ramifié, ou un mélange contenant une quantité prépondérante dudit composé (VI), avec un composé organométallique de formule $R^2MgX$, dans laquelle X représente un atome d'halogène et $R^2$ est défini comme à la formule (I).

Les composés de formule (VI) utilisés comme produits de départ dans le procédé selon l'invention sont des composés nouveaux qui peuvent être préparés à partir de la 2,2,3,6-tétraméthyl-1-cyclohexanone (mélange contenant essentiellement l'isomère trans), selon la méthode originale illustrée à l'aide du schéma que voici :

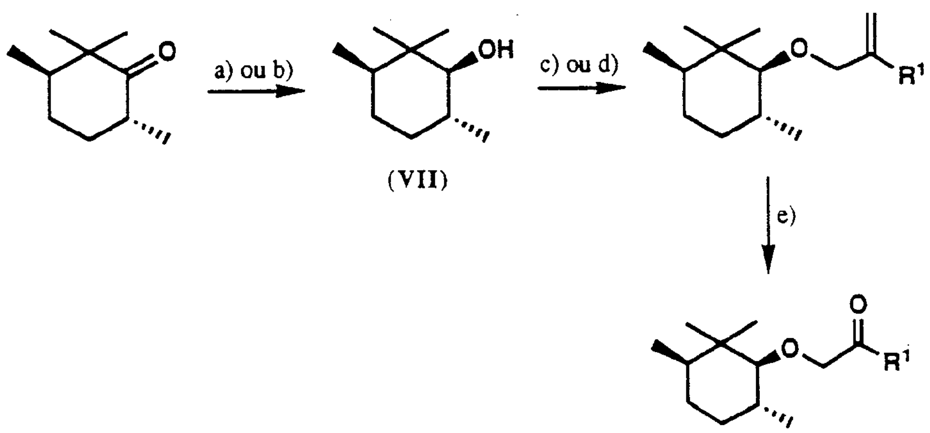


(VII)

(VI)

X = Cl ou Br

$R^1$ défini comme ci-dessus

a) Na, isopropanol, toluène

b) Li, $NH_3$, isopropanol

c) butyllithium, THF,

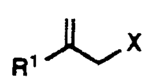

d) Li, styrène, THF, toluène,

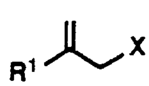

e) $O_3$, MeOH, $Na_2SO_3$, $H_2O$

La 2,2,3,6-tétraméthyl-1-cyclohexanone, essentiellement sous sa forme isomérique trans, utilisée comme produit de départ dans le procédé décrit ci-dessus, peut être préparée conformément à la méthode décrite par G. Schäppi et C. F. Seidel [voir Helv. Chim. Acta, 30, 2199 (1947)].

La première étape de ce procédé consiste en la réduction de la cétone indiquée. Le produit de cette réaction

est un mélange contenant essentiellement le 2,2,c-3,t-6-tétraméthyl-r-1-cyclohexanol (VII), ainsi que des quantités mineures des trois stéréoisomères de formules

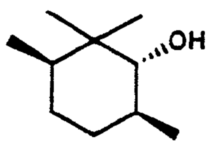
(VIII)
,
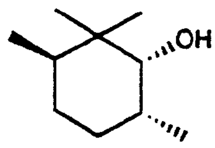
(IX)
et
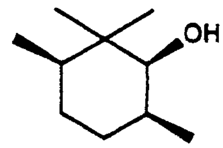
(X)

Les conditions de la réaction ont été optimisées de façon à obtenir un mélange aussi riche que possible en alcool (VII) et ayant un contenu total en stéréoisomères cis (IX) et (X) très bas, afin de garantir la pureté stéréochimique du composé de formule (VI) utilisé comme produit de départ dans le procédé selon l'invention. C'est ainsi que nous avons découvert que ce but était atteint de la façon la plus favorable lorsqu'on ajoutait une solution de la cétone de départ dans l'isopropanol à une solution de lithium dans de l'ammoniac au reflux [lettre b) du schéma ci-dessus], suivant une méthode analogue à celle qui a été décrite par J. W. Huffman et al. dans J. Am. Chem. Soc., 90, 6486 (1968).

Les conditions détaillées de cette réaction seront décrites dans les exemples de préparation présentés plus loin. Cette méthode a permis d'obtenir un mélange contenant environ 79% de l'alcool (VII), 17% de l'alcool (VIII) et respectivement 1 et 3% des stéréoisomères cis, les alcools (IX) et (X). L'alcool (VII) désiré a été obtenu à l'aide de deux recristallisations successives de ce mélange dans le pentane. Cependant, étant donné le faible contenu de ce mélange en isomères (IX) et (X) (environ 4%), il peut également être utilisé tel quel dans l'étape suivante du procédé illustré dans le schéma.

Les conditions de réduction de la 2,2,3,6-tétraméthyl-1-cyclohexanone décrites sous lettre a) de ce schéma ont permis d'obtenir un mélange composé comme suit : 73% (VII) + 18% (VIII) + 5% (IX) + 4% (X).

Lorsqu'on a utilisé des conditions classiques de réduction de cétones alicycliques, on a obtenu des mélanges d'alcools beaucoup plus riches en isomères cis. Par exemple, l'utilisation de $LiAlH_4$, en présence d'éther éthylique, utilisant le même produit de départ, a conduit à un mélange d'alcools contenant 51% de (VII), 28% de (IX) et 21% de (X). De même, l'emploi de $NaBH_4$ dans l'éthanol, ou d'hydrogène en présence d'oxyde de platine et acide acétique, ont produit des mélanges contenant des quantités d'isomères (IX) + (X) comparables au contenu du mélange en alcool (VII).

L'allylation de l'alcool (VII) ou du mélange riche en alcools (VII) et (VIII) se réalise avec un meilleur rendement lorsqu'elle est effectuée par une base forte telle que le n-butyllithium, ou mieux encore, du lithium, en présence de tétrahydrofuranne et, dans le deuxième cas, de styrène.

L'ozonolyse du dérivé allyloxy obtenu dans la réaction précédente, suivie d'une extraction réductrice de l'ozonide formé, à l'aide de $Na_2SO_3$ aqueux, permet d'obtenir le composé (VI) désiré. Lorsque le produit de départ de la réaction d'allylation est l'un des mélanges cités riches en alcools (VII) et (VIII), on obtiendra comme produit de l'ozonolyse un mélange riche en composé (VI), accompagné de quantités inférieures de son isomère dérivé de l'alcool trans (VIII). Etant donné que les réactions d'allylation et ozonolyse se réalisent avec rétention de la stéréochimie du produit de départ, ces mélanges riches en l'un des composés (VI) contiendront au moins 70% en poids de ce dernier, et moins de 10% en poids d'isomères cis correspondants, par rapport au poids du mélange.

Selon l'invention, les composés de formule (I) sont préparés par réaction d'un composé de formule (VI), obtenu suivant la méthode décrite ci-dessus, avec un réactif de Grignard de formule $R^2MgX$ définie précédemment.

Alternativement, on utilisera comme produit de départ du procédé selon l'invention, un mélange riche en composé de formule (VI) tel que défini ci-dessus, pour obtenir un mélange riche en stéréoisomères de formule (I) et (III), qui pourra être enrichi en composé de formule (I) ou fournir ce dernier pur, à l'aide des techniques usuelles de purification telles que la chromatographie en phase gazeuse. De préférence, le mélange utilisé en tant que produit de départ contiendra au moins environ 75% en poids de composé de formule (VI) et pas plus d'environ 10% en poids de l'ensemble des stéréoisomères cis de ce composé, dérivés des alcools (IX) et (X), le reste du mélange étant constitué par l'autre stéréoisomère trans dérivé de l'alcool (VIII).

Le procédé selon l'invention est décrit plus en détail dans les exemples de préparation suivants, dans lesquels les températures sont indiquées en degrés centigrades et les abréviations ont le sens usuel dans l'art. Des exemples d'application des composés de formule (I) et des mélanges selon l'invention dans des compositions parfumantes et produits parfumés sont également décrits ci-après.

Exemples 1 à 22

Méthode générale des réactions de Grignard

Dans un ballon muni d'une agitation mécanique, on a chargé 0,12 mole de magnésium et 25 ml de THF (tétrahydrofuranne) ou d'éther absolu. La réaction a été amorcée avec un petit cristal d'iode et quelques gouttes d'iodure de méthyle. On a introduit goutte à goutte une solution de 0,12 mole de l'halogénure approprié dans 25 ml de THF ou éther absolu (la température est montée à reflux). Après 30 min d'agitation jusqu'à disparition du magnésium, on a introduit goutte à goutte 0,1 mole de l'aldéhyde, respectivement de la cétone choisie (la température est montée à reflux). On a agité 1 h, respectivement 2 h, à température ambiante, versé le mélange de la réaction sur glace et $NH_4Cl$, extrait à l'éther, lavé avec $NH_4Cl$, saturé à neutralité, séché et concentré. Selon cette méthode, on a préparé les composés suivants :

1) 1-(2,2,c-3,t-6-tétraméthyl-r-1-cyclohexyloxy)-2-pentanol

à partir de 79,7 g (0,402 mole) de l'aldéhyde préparé selon le procédé indiqué ci-après et de 66 ml (0,72 mole) de bromure de propyle dans le THF. Après distillation sur résidu et ensuite à la colonne à bande tournante, on a isolé le produit désiré à 96% pur, sous forme d'un mélange contenant environ 80% de l'isomère susmentionné et 20% de 1-(2,2,t-3,t-6-tétraméthyl-r-1-cyclohexyloxy)-2-pentanol.
Rend. 59% ; P. éb. 55°/4,8 Pa.
Données analytiques du 1-(2,2,c-3,t-6-tétraméthyl-r-1-cyclohexyloxy)-2-pentanol :

| | |
|---|---|
| IR: | 3450 (-OH) ; 1100 (-O-) $cm^{-1}$ |
| RMN(360MHz, $^1H$) : | 0,74-0,98(m,15H) ; 2,42(d,1H,J=10,8Hz) ; 3,33-3,62(m,2H) ; 3,79(m,1H) delta ppm ; |
| SM : | $M^+$ = 242(43) ; m/e : 157(30), 138(96), 123(38), 109(15), 96(20), 87(19), 83(39), 69(37), 55(59), 41(100). |
| Qualification olfactive : | voir Tableau I. |

2) 1-(2,2,c-3,t-6-tétraméthyl-r-1-cyclohexyloxy)-4-pentén-2-ol

à partir de 5,0 g (25 mmole) de l'aldéhyde préparé selon le procédé indiqué ci-après, dans 8,2 g de THF, et de bromure d'allyle. La distillation dans un four à boules sur résidu a fourni 3,6 g de produit à 44% pur. Ce produit a été chromatographié sur $SiO_2$, en utilisant un mélange éther de pétrole/éther sulfurique 5:1 comme éluant, pour donner 0,6 g de produit à 98% pur, sous forme d'un mélange contenant 85% de l'isomère désiré et 15% de 1-(2,2,t-3,t-6-tétraméthyl-r-1-cyclohexyloxy)-4-pentén-2-ol.
Rend. 18%.
Données analytiques du 1-(2,2,c-3,t-6-tétraméthyl-r-1-cyclohexyloxy)-4-pentén-2-ol :

| | |
|---|---|
| IR : | 3450 (-OH) $cm^{-1}$ |
| RMN(360MHz, $^1H$) : | 0,74(d,3H,J=7Hz) ; 0,82(d,3H,J=7Hz) ; 0,96(s,6H) ; 2,43(d,1H,J=11Hz) ; 3,40-3,62(m,2H) ; 3,85(m,1H) ; 5,8(d,1H,J=9Hz) ; 5,25(d,1H,J=17Hz) ; 5,85(m,1H) delta ppm ; |
| SM : | $M^+$ = 240(6) ; m/e : 155(16), 38(60), 123(28), 96(40), 83(100), 69(70). |
| Qualification olfactive : | voir Tableau I. |

3) 1-(2,2,c-3,t-6-tétraméthyl-r-1-cyclohexyloxy)-2-butanol

à partir de 8,0 g (40 mmole) de l'aldéhyde préparé selon le procédé indiqué ci-après, dans 10,9 g de THF, et de bromure d'éthyle. Après distillation sur colonne Vigreux, on a obtenu 5,35 g de produit à 80% pur. Une purification supplémentaire a fourni un produit pur sous forme d'un mélange contenant 85% de l'isomère désiré et 15% de 1-(2,2,t-3,t-6-tétraméthyl-r-1-cyclohexyloxy)-2-butanol.
Rend. 46% ; P. éb. 61°/4,2 Pa.
Données analytiques du 1-(2,2,c-3,t-6-tétraméthyl-r-1-cyclohexyloxy)-2-butanol :

| | |
|---|---|
| IR: | 3450 (-OH) ; 1090 (-O-) $cm^{-1}$ |
| RMN (360MHz,$^1H$) : | 0,74(d,3H) ; 0,83(d,3H) ; 0,96(s,6H) ; 0,97(t,3H) ; 2,43(d,1H,J=environ 10,8Hz) ; 3,36 à 3,63(m,2H) ; 3,70(m,1H) delta ppm ; |
| SM : | $M^+$ = 228(43) ; m/e : 156(3), 143(31), 138(100), 123(39), 109(21), 96(32), 83(62), 73(51), 55(85), 41(67). |
| Qualification olfactive : | voir Tableau I. |

4) 2-méthyl-1-(2,2,c-3,t-6-tétraméthyl-r-1-cyclohexyloxy)-2-butanol

à partir de 8,0 g (38 mmole) de la cétone préparée selon le procédé indiqué ci-après, dans 8,8 g de THF, et bromure d'éthyle. Après distillation à la colonne à bande tournante, on a isolé 4,75 g de produit à 88% pur, qui a été encore purifié pour fournir un mélange à 99% pur contenant 93% de l'isomère désiré et 7% de 2-méthyl-1-(2,2,t-3,t-6-tétraméthyl-r-1-cyclohexyloxy)-2-butanol.

Rend. 46% ; P. éb. 45°/5 Pa.

| | |
|---|---|
| IR : | 3450, 2950, 1460, 1100 $cm^{-1}$ ; |
| RMN (360MHz,$^1$H) : | isomère majoritaire : 0,82(d,3H,J=7Hz) ; 0,96(s,3H) ; 1,17(s,3H) ; 2,43(d,1H,J=11Hz) ; 3,43(AB,2H,$J_1$=9Hz, $J_2$=32Hz) delta ppm ; |
| RMN (360MHz,$^1$H) : | isomère minoritaire : 2,78(d,1H,J=11Hz) ; 3,38(AB,2H, $J_1$=8Hz,$J_2$=36Hz) delta ppm ; |
| SM (isomère majoritaire) : | $M^+$ = 242(1) ; m/e : 170(20), 139(28), 83(100), 73(81), 69(53), 55(45) ; |
| SM (isomère minoritaire) : | $M^+$ = 242(2) ; m/e : 170(20), 139(28), 123(23), 83(100), 73(80), 69(61), 55(53). |
| Qualification olfactive : | voir Tableau I. |

5) 4-méthyl-1-(2,2,c-3,t-6-tétraméthyl-r-1-cyclohexyloxy)-2-pentanol

à partir de l'aldéhyde préparé selon le procédé indiqué ci-après(73,0 g, 0,37 mole), dans le THF (88,2 g), et de bromure d'isobutyle, suivant la méthode de Grignard générale.

Ce composé a également été préparé par hydrogénation du 4-méthyl-1-(2,2,c-3,t-6-tétraméthyl-r-1-cyclohexyloxy)-4-pentén-2-ol, dont la préparation est décrite plus loin [voir 11)], selon la méthode suivante : dans un ballon à 1 col, muni d'une tête d'hydrogénation, on a hydrogéné pendant 18 h à température ambiante un mélange de 1,9 g (7,5 mmole) du penténol susmentionné, 20 ml d'éthanol et 500 mg de Nickel de Raney (prise pratique d'hydrogène : 250 ml). Après filtration du mélange de la réaction sur Célite (marque enregistrée) et concentration, on a obtenu 2,0 g de produit brut qui a été finement distillé dans un four à boules. On a isolé 1,5 g de produit à 95% pur, sous forme d'un mélange contenant 82% de l'isomère désiré et 18% de 4-méthyl-1-(2,2,c-3,c-6-tétraméthyl-r-1-cyclohexyloxy)-2-pentanol.

Rend. 73% ; P. éb. 80-85°/5 Pa.

Données analytiques du 4-méthyl-1-(2,2,c-3,t-6-tétraméthyl-r-1-cyclohexyloxy)-2-pentanol :

| | |
|---|---|
| IR : | 3450 (-OH), 1090 (-O-) $cm^{-1}$ ; |
| RMN (360MHz,$^1$H) : | 0,74(d,3H) ; 0,82(d,3H) ; 0,93(s,3H) ; 0,95(s,3H) ; 0,98(d,6H) ; 2,42(d,1H,J=environ 10,8Hz) ; 3,33 à 3,60(m,2H) ; 3,87(m,1H) delta ppm ; |
| SM : | $M^+$ = 256(56) ; m/e : 171(21), 156(5), 138(100), 123(40), 101(17), 96(35), 83(81), 69(52), 55(79), 43(87). |
| Qualification olfactive : | voir Tableau I. |

6) 2-méthyl-1-(2,2,c-3,t-6-tétraméthyl-r-1-cyclohexyloxy)-2-propanol

à partir de 40,0 g (0,19 mole) de la cétone préparée selon le procédé indiqué ci-après, dans 43,7 g d'éther, avec iodure de méthyle. Après distillation sur résidu, on a isolé 35,6 g de produit à 83% pur. Une purification supplémentaire a fourni un produit à 98% pur, sous forme d'un mélange contenant 79% de l'isomère désiré, 14% de 2-méthyl-1-(2,2,t-3,t-6-tétraméthyl-r-1-cyclohexyloxy)-2-propanol et 7% de 2-méthyl-1-(2,2,t-3,c-6-tétraméthyl-r-1-cyclohexyloxy)-2-propanol.

Rend. 68% ; P. éb. 72°/5 Pa.

IR : 3400, 2900, 1450, 1090 $cm^{-1}$ ;

Isomère c-3,t-6 (79%)

| | |
|---|---|
| RMN (360MHz,$^1$H) : | 0,76(s,3H) ; 0,83(d,3H,J=7Hz) ; 0,95(d,3H,J=7Hz) ; 0,96(s,3H) ; 1,21(s,3H) ; 1,24(s,3H) ; 2,43(d,1H,J=11Hz) ; 3,4(AB,2H,$J_1$=7Hz, $J_2$=23Hz) delta ppm ; |
| SM : | $M^+$ = 228(14) ; m/e : 170(52), 139(52), 123(35), 96(35), 83(98), 69(59), 59(80), 55(100), 43(92). |

Isomère t-3,t-6 (14%)

RMN(360MHz,$^1$H) : 2,79(d,1H,J=11Hz) ; 3,36(AB,2H,$J_1$=7Hz,$J_2$=16Hz) delta ppm ;

SM : $M^+$ = 228(30) ; m/e : 170(31), 139(59), 123(39), 83(100), 69(66), 55(86), 51(68).

Isomère t-3,c-6 (7%)

SM : $M^+$ = 228(15), 170(35), 139(46), 123(34), 83(100), 69(68), 59(70), 55(91), 41(74).
Qualification olfactive : voir Tableau I.

7) 2-méthyl-1-(2,2,c-3,t-6-tétraméthyl-r-1-cyclohexyloxy)-4-pentén-2-ol

à partir de bromure d'allyle et de 8,0 g (38 mole) de la cétone préparée selon le procédé indiqué ci-après, dans du THF (9,8 g). La distillation en colonne à bande tournante a fourni un produit à 93% pur que l'on a encore purifié à 99%. On a obtenu un mélange contenant 98% de l'isomère désiré et 2% de 2-méthyl-1-(2,2,t-3,t-6-tétraméthyl-r-1-cyclohexyloxy)-4-pentén-2-ol.
Rend. 60% ; P. éb. 57°/5 Pa.
IR : 3450, 2950, 1640, 1460, 1100 $cm^{-1}$ ;

Isomère majoritaire (98%)

RMN (360MHz,$^1$H) : 0,75(s,3H) ; 0,82(d,3H,J=7Hz) ; 0,95(d,3H,J=7Hz) ; 0,97(s,3H) ; 1,2(s,3H) ; 2,3(m,2H) ; 2,43(d,1H,J=11Hz) ; 3,4(AB,2H,$J_1$=7Hz,$J_2$=46Hz) ; 5,09(d,2H,J=15Hz) ; 5,87(m,1H) delta ppm ;
SM : $M^+$ = 254(1) ; m/e : 170(14), 139(41), 83(100), 69(50), 57(38), 43(48).

Isomère minoritaire (2%)

RMN (360MHz,$^1$H) : 2,78(d,1H,J=11Hz) delta ppm ;
SM : $M^+$ = 254(0) ; m/e : 170(17), 139(47), 83(100), 69(53), 55(43), 43(55).
Qualification olfactive : voir Tableau I.

8) 1-(2,2,c-3,t-6-tétraméthyl-r-1-cyclohexyloxy)-3-pentén-2-ol

à partir de 1-bromopropène et de 10,0 g (50 mmole) de l'aldéhyde préparé selon le procédé indiqué ci-après, dans le THF (10,8 g). Après distillation sur résidu au four à boules puis finement à la colonne à bande tournante, on a isolé 6,6 g de produit à 97% pur, qui a encore été purifié pour fournir le penténol désiré 99% pur.
Rend. 53% ; P. éb. 64°/2,5 Pa.
RMN (360 MHz,$^1$H) : 0,75 et 0,76(2s, 1,5 et 1,5H) ; 0,83(d,3H,J=7Hz) ; 0,97(d,3H,J=7Hz) ; 0,98 et 0,99(2s, 1,5 et 1,5H) ; 1,69 et 1,71(2t,1,5 et 1,5H,J=3Hz) ; 2,43(d,1H,J=11Hz) ; 3,48(m,2H) ; 4,66(m,1H) ; 5,38(m,1H) ; 5,63(m,1H) delta ppm ;
SM : $M^+$ = 240(0) ; m/e : 139(30), 83(100), 69(58), 55(34), 43(16).
Qualification olfactive : voir Tableau I.

9)1-(2,2,c-3,t-6-tétraméthyl-r-1-cyclohexyloxy)-2-hexanol

à partir de bromure de n-butyle et 3,1 g (15,6 mmole) de l'aldéhyde préparé selon le procédé indiqué ci-après, dans le THF absolu (3,2 g, 33% pur). Après purification sur colonne de $SiO_2$ avec un mélange cyclohexane-/éther acétique 95:5 comme éluant, on a isolé 300 mg d'un mélange 91% pur contenant 85% de l'isomère désiré et 15% de 1-(2,2,t-3,t-6-tétraméthyl-r-1-cyclohexyloxy)-2-hexanol. Une synthèse ultérieure a permis d'obtenir un mélange 99% pur, contenant 95% de l'isomère désiré et 5% de l'autre isomère cité.
Rend. environ 26%.
Données analytiques du 1-(2,2,c-3,t-6-tétraméthyl-r-1-cyclohexyloxy)-2-hexanol:
IR : 3450 (-OH), 1090 (-O-) $cm^{-1}$ ;
RMN (360MHz,$^1$H) : 0,74(d,3H) ; 0,82(d,3H) ; 0,91(t,3H) ; 0,96(s,6H) ; 2,43(d,1H,J=environ 10,8Hz) ; 3,36 à 3,62(m,2H) ; 3,78(m,1H) delta ppm ;
SM : $M^+$ = 256(47) ; m/e : 171(19), 156(4), 138(100), 123(38), 109(18), 96(33), 83(71), 69(40), 55(62), 41(81).
Qualification olfactive : boisé, camphré.

10) 3-méthyl-1-(2,2,c-3,t-6-tétraméthyl-r-1-cyclohexyloxy)-2-butanol

à partir d'iodure d'isobutyle et de 6,2 g (31 mmole) de l'aldéhyde préparé selon le procédé indiqué ci-après dans 6,5 g d'éther absolu. La distillation sur une colonne de 25 cm a permis d'obtenir 3,35 g d'un produit à 52% pur contenant 83% de l'alcool désiré, 14% de 3-méthyl-1-(2,2,t-3,t-6-tétraméthyl-r-1-cyclohexyloxy)-2-butanol et 3% de 3-méthyl-1-(2,2,t-3,c-6-tétraméthyl-r-1-cyclohexyloxy)-2-butanol.
La purification supplémentaire de ce produit, par chromatographie sur colonne de $SiO_2$, avec un mélange cyclohexane/éther acétique 95:5 comme éluant, a permis d'isoler un mélange à 98% pur, contenant 85% de 3-méthyl-1-(2,2,c-3,t-6-tétraméthyl-r-1-cyclohexyloxy)-2-butanol et 15% de 3-méthyl-1-(2,2,t-3,t-6-tétraméthyl-r-1-cyclohexyloxy)-2-butanol.
Rend. 23%.
Données analytiques du 3-méthyl-1-(2,2,c-3,t-6-tétraméthyl-r-1-cyclohexy-loxy)-2-butanol :

| | |
|---|---|
| IR : | 3450 (-OH), 1090 (-O-) $cm^{-1}$; |
| RMN (360MHz,$^1$H) : | 0,73(d,3H) ; 0,82(d,3H) ; 0,90(d,6H) ; 0,96(s,3H) ; 0,98(s,3H) ; 2,42(d,1H,J=environ 10,8Hz) ; 3,42 à 3,56(m,2H) ; 3,63(m,1H) delta ppm ; |
| SM : | $M^+$ = 242(48) ; m/e = 199(2), 181(1), 170(3), 157(22), 138(100), 123(40), 109(21), 96(30), 87(39), 83(63), 69(58), 55(69), 43(81). |
| Qualification olfactive : | boisé. |

11) 4-méthyl-1-(2,2,c-3,t-6-tétraméthyl-r-1-cyclohexyloxy)-4-pentén-2-ol

à partir de chlorure de 2-méthylallyle et 17,0 g (86 mmole) de l'aldéhyde préparé selon le procédé indiqué ci-après, dans THF absolu (24,2 g). Après distillation sur colonne Vigreux, on a obtenu 3,6 g de produit à 71% pur, qui a été encore purifié par chromatographie sur colonne de $SiO_2$, avec un mélange cyclohexane/éther acétique 95:5 comme éluant. On a obtenu ainsi un mélange à 95% pur, contenant 85% de 4-méthyl-1-(2,2,c-3,t-6-tétraméthyl-r-1-cyclohexyloxy)-4-pentén-2-ol et 15% de 4-méthyl-1-(2,2,t-3,t-6-tétraméthyl-r-1-cyclohexyloxy)-4-pentén-2-ol.
Rend. 11% ; P. éb. 88°/4,6 Pa.
Données analytiques du 4-méthyl-1-(2,2,c-3,t-6-tétraméthyl-r-1-cyclohexy-loxy)-4-pentén-2-ol :

| | |
|---|---|
| IR : | 3450 (-OH), 1090 (-O-) $cm^{-1}$ ; |
| RMN (360MHz,$^1$H) : | 0,75(d,3H) ; 0,82(d,3H) ; 0,96(s,3H) ; 0,98(s,3H) ; 1,78(s,3H) ; 2,20(m,2H) ; 2,43(d,1H,J=environ 10,8Hz) ; 3,41 à 3,62(m,2H) ; 3,97(m,1H) ; 4,82(d,2H,J=environ 16,2Hz) delta ppm ; |
| SM : | $M^+$ = 254(7) ; m/e : 198(7), 169(1), 156(2), 139(51), 123(21), 114(7), 109(12), 101(30), 96(21), 83(61), 69(31), 55(50), 43(100). |
| Qualification olfactive : | boisé, légèrement ambré. |

12) 1-(2,2,c-3,t-6-tétraméthyl-r-1-cyclohexyloxy)-3-butén-2-ol

à partir de bromure de vinyle et 9,75 g (49 mmole) de l'aldéhyde préparé selon le procédé indiqué ci-après, dans 14,8 g de THF. La purification par distillation au four à boules sur résidu, puis finement sur une colonne à bande tournante, a permis d'obtenir 1,3 g de produit à 96% pur. Une purification supplémentaire a fourni un mélange 99% pur, contenant 85% de l'alcool désiré et 15% de 1-(2,2,t-3,t-6-tétraméthyl-r-1-cyclohexyloxy)-3-butén-2-ol.
Rend. 11% ; P. éb. 55°/6,7 Pa.
Données analytiques du 1-(2,2,c-3,t-6-tétraméthyl-r-1-cyclohexyloxy)-3-butén-2-ol :

| | |
|---|---|
| IR : | 3450 (-OH), 2900 ($-CH_2-$), 1100 (-O-) $cm^{-1}$ ; |
| RMN (360MHz,$^1$H) : | 0,83(d,3H,J=7Hz) ; 0,97(d,3H,J=7Hz) ; 0,97(s,6H) ; 2,44(d,1H,J=11Hz) ; 3,40-3,66(m,2H) ; 4,31(m,1H) ; 5,19(d,1H,J=10Hz) ; 5,36(d,1H,J=16Hz) ; 5,85(m,1H) delta ppm ; |
| SM : | $M^+$ = 226 ; m/e : 154(5), 139(55), 123(35), 109(8), 95(36), 91(7), 83(100), 69(40), 55(42), 41(29). |
| Qualification olfactive : | boisé, épicé, ambré. |

13) 1-(2,2,c-3,t-6-tétraméthyl-r-1-cyclohexyloxy)-2-propanol

à partir d'iodure de méthyle et de 17,4 g (88 mmole) de l'aldéhyde préparé selon le procédé indiqué ci-après, dans 19,7 g d'éther/THF absolu. Après distillation fine à la colonne à bande tournante, on a obtenu 7,1 g de

produit 92% pur. Une purification supplémentaire a permis d'isoler un mélange à 95% pur contenant 86% de l'alcool désiré, 9% de 1-(2,2,t-3,t-6-tétraméthyl-r-1-cyclohexyloxy)-2-propanol et 5% de 1-(2,2,c-3,c-6-tétraméthyl-r-1-cyclohexyloxy)-2-propanol.

Rend. 34% ; P. éb. 45°/2,3 Pa.

Données analytiques du 1-(2,2,c-3,t-6-tétraméthyl-r-1-cyclohexyloxy)-2-propanol :

| | |
|---|---|
| IR : | 3350 (-OH), 1080 (-O-) $cm^{-1}$ ; |
| RMN (360MHz,$^1H$) : | 0,74(d,3H) ; 0,82(d,3H) ; 0,96(s,6H) ; 1,14(dxd,3H) ; 2,43(d,1H,J=environ 10,8Hz) ; 3,29 à 3,58(m,2H) ; 3,92(m,1H) delta ppm ; |
| SM : | $M^+$ = 214(21) ; m/e : 171(1), 156(1), 138(100), 129(47), 123(51), 113(4), 109(31), 96(61), 83(98), 71(92), 59(93), 55(82), 41(72). |
| Qualification olfactive : | boisé, celluloïde, camphré. |

14) <u>2-méthyl-1-(2,2,c-3,t-6-tétraméthyl-r-1-cyclohexyloxy)-2-pentanol</u>

à partir de bromure de propyle et de 6,4 g (30 mmole) de la cétone préparée selon le procédé indiqué ci-après, dans 7,9 g de THF. On a isolé, par distillation fine à la colonne à bande tournante, 2,9 g de l'alcool désiré à 90% pur. Une nouvelle purification a fourni cet alcool à 97% pur.

Rend. 33% ; P. éb. 47°/2 Pa.

| | |
|---|---|
| IR : | 3450, 2950, 1460, 1100 $cm^{-1}$ ; |
| RMN (360MHz,$^1H$) : | 0,75(s,3H) ; 0,83(d,3H,J=7Hz) ; 0,95(d,3H,J=7Hz) ; 0,96(s,3H) ; 1,19(s,3H) ; 2,42(d,1H,J=11Hz) ; 3,4(AB,2H,$J_1$=8Hz,$J_2$=40Hz) delta ppm ; |
| SM : | $M^+$ = 256(0,5) ; m/e : 170(34), 139(36), 87(77), 83(100), 69(43), 55(45), 43(29). |
| Qualification olfactive : | ambré, boisé, camphré. |

15) <u>2,4-diméthyl-1-(2,2,c-3,t-6-tétraméthyl-r-1-cyclohexyloxy)-2-pentanol</u>

a) Ce composé a été préparé par la méthode générale de Grignard à partir d'iodure de méthyle et de 3,5 g (14 mmole) de 4-méthyl-1-(2,2,c-3,t-6-tétraméthyl-1-cyclohexyloxy)-2-pentanone (préparée comme décrit ci-dessous) dans l'éther (3,85 g). On a distillé au four à boules sur résidu et chromatographié sur colonne de $SiO_2$, avec un mélange cyclohexane/éther acétique 95:5 comme éluant. On a isolé ainsi 3,15 g d'un mélange 96% pur, contenant 96% du pentanol désiré et 4% de 2,4-diméthyl-1-(2,2,t-3,t-6-tétraméthyl-r-1-cyclohexyloxy)-2-pentanol.

Rend. 78%.

IR : 3600, 2940, 1460, 1100 $cm^1$ ;

<u>Isomère majoritaire (96%)</u>

| | |
|---|---|
| RMN (360MHz,$^1H$) : | 0,75 et 0,76(2s, 1,5 et 1,5H) ; 0,82(d,3H,J=7Hz) ; 0,95(d,3H,J=7Hz) ; 0,97(s,3H) ; 1,2 et 1,23(2s, 1,5 et 1,5H) ; 2,42(d,1H,J=11Hz) ; 3,39(AB et delta, 1 et 1H, $J_1$=7Hz,$J_2$=36Hz) delta ppm ; |
| SM : | $M^+$ = 270(0) ; m/e : 170(19), 139(22), 101(68), 83(100), 69(50), 55(57), 43(40). |

<u>Isomère minoritaire (4%)</u>

| | |
|---|---|
| RMN (360MHz,$^1H$) : | 2,78(d,1H,J=11Hz) delta ppm ; |
| SM : | $M^+$= 270(0) ; m/e : 170(18), 139(22), 101(59), 83(100). |
| Qualification olfactive : | boisé, nuance fromage. |

b) <u>4-méthyl-1-(2,2,c-3,t-6-tétraméthyl-r-1-cyclohexyloxy)-2-pentanone</u> Dans un ballon à 4 cols muni d'une agitation mécanique, on a chargé 200 ml d'acide acétique conc. et 25 ml d'eau. On a ajouté 21,6 g (216 mmole) de trioxyde de chrome et refroidi à 0° (glace et sel). Ensuite, on a introduit goutte à goutte 13,8 g (54 mmole) de 4-méthyl-1-(2,2,c-3,t-6-tétraméthyl-r-1-cyclohexyloxy)-2-pentanol [voir 5)] sans dépasser 0°, et agité encore pendant 30 min. On a ajouté du NaOH à 30% sans dépasser 20°C pour marquer alcalin, extrait à l'éther, lavé à l'eau à neutralité, séché et concentré : 12,05 g de produit brut ont été obtenus. Après avoir distillé finement à la colonne à bande tournante, on a isolé ainsi 4,5 g de cétone à 93% pure sous forme d'un mélange contenant 97% de l'isomère désiré et 3% de 4-méthyl-1-(2,2,t-3,t-6-tétraméthyl-r-1-cyclohexyloxy)-2-pentanone.

Rend. 30% ; P. éb. 53°/3 Pa.

IR : 2960, 1720, 1460, 1365, 1110 $cm^{-1}$ ;

<u>Isomère majoritaire (97%)</u>

| | |
|---|---|
| RMN (360MHz,$^1H$) : | 0,8(s,3H) ; 0,83(d,3H,J=7Hz) ; 0,94(d,3H,J=7Hz) ; 0,95(s,3H) ; 2,42(d,1H,J=11Hz) ; 4,13(AB,2H,$J_1$=15Hz,$J_2$=22Hz) delta ppm ; |
| SM : | $M^+$ = 254(0) ; m/e : 155(9), 139(26), 83(100), 69(32), 57(36), 41(21). |

Isomère minoritaire (3%)

RMN (360MHz,$^1$H) : 2,77(d,1H,J=11Hz) delta ppm ;
SM : $M^+$ = 254(0) ; m/e : 139(20), 83(100), 69(39), 57(38), 41(22).

16) 2-méthyl-1-(2,2,c-3,t-6-tétraméthyl-r-1-cyclohexyloxy)-3-butén-2-ol

à partir de bromure de vinyle et 6,55 g (31 mmole) de la cétone préparée selon le procédé indiqué ci-après, dans le THF (7,7 g). Après avoir distillé finement à la colonne à bande tournante, on a isolé 4,55 g de produit à 91% pur, qui a été purifié encore pour fournir un mélange à 95% pur contenant 80% du buténol susmentionné et 20% de 2-méthyl-1-(2,2,t-3,t-6-tétraméthyl-r-1-cyclohexyloxy)-3-butén-2-ol.
Rend. 56% ; P. éb. 53°/2 Pa ;
IR : 3450, 2950, 1450, 1100 $cm^{-1}$ ;

Isomère majoritaire (80%)

RMN (360MHz,$^1$H) : 0,72 et 0,75(s, 1,2 et 1,8H) ; 0,82 et 0,83(d, 1,2 et 1,8H,J=7Hz) ; 0,94(d,3H,J=7Hz) ; 0,95 et 0,97(s, 1,2 et 1,8H) ; 1,26 et 1,30(s, 1,2 et 1,8H) ; 2,42 et 2,43(d, 0,4 et 0,6H,J=11Hz) ; 3,45(AB,2H,$J_1$=7Hz,$J_2$=30Hz) ; 5,1(d,1H,J=10Hz) ; 5,31(d,1H,J=16Hz) ; 5,94(m,1H) delta ppm ;
SM : $M^+$ = 240(0) ; m/e : 139(28), 123(20), 83(100), 69(44), 55(47), 43(36).

Isomère minoritaire (20%)

RMN (360MHz,$^1$H) : 2,78(d,1H,J=11Hz) ; 3,41(AB,2H,$J_1$=12Hz,$J_2$=16Hz) delta ppm ;
SM : $M^+$ = 240(0) ; m/e : 139(28), 123(22), 83(100), 69(44), 55(53), 43(40).
Qualification olfactive : boisé, faible.

17) 2,3-diméthyl-1-(2,2,c-3,t-6-tétraméthyl-r-1-cyclohexyloxy)-2-butanol

à partir de 2-bromopropane et de 6,5 g (31 mmole) de la cétone préparée selon le procédé indiqué ci-après, dans le THF (7,2 g). On a distillé finement à la colonne à bande tournante pour obtenir 2,05 g de produit à 75% pur. Une purification supplémentaire par chromatographie sur $SiO_2$, en utilisant comme éluant un mélange cyclohexane/éther acétique 95:5, a permis d'obtenir l'alcool désiré à 99% pur.
Rend. 20% ; P. éb. 47°/2 Pa.
IR : 3570, 2950, 1460, 1100 $cm^{-1}$ ;
RMN (360MHz,$^1$H) : 0,74 et 0,76(2s, 1,5 et 1,5H) ; 0,82(d,3H,J=7Hz) ; 0,88(2d,6H,J=7Hz) ; 0,96(d,3H,J=7Hz) ; 0,97(s,3H) ; 1,07 et 1,11(2s, 1,5 et 1,5H) ; 1,85(hept,1H,J=7Hz) ; 2,43(d,1H,J=11Hz) ; 3,46(2AB, 1 et 1H,$J_1$=7Hz, $J_2$=58Hz, $J_{11}$=7Hz, $J_{12}$=22Hz) delta ppm ;
SM : $M^+$ = 256(0) ; m/e : 170(21), 139(29), 123(21), 87(79), 83(100), 69(46), 55(36), 43(23).

18) 3-méthyl-1-(2,2,c-3,t-6-tétraméthyl-1-cyclohexyloxy)-3-butén-2-ol

à partir de 2-bromopropène et de 10,0 g (50 mmole) de l'aldéhyde préparé selon le procédé indiqué ci-après, dans le THF (12,0 g). On a distillé sur résidu au four à boules (9,2 g de produit) puis finement à la colonne à bande tournante. On a isolé ainsi 6,85 g du buténol désiré à 88% pur. Une nouvelle purification par chromatographie sur colonne a permis d'obtenir le produit 98% pur.
Rend. 49% ; P. éb. 42°/3 Pa.
IR : 3460, 2960, 1650, 1450, 1100 $cm^{-1}$ ;
RMN (360MHz,$^1$H) : 0,74 et 0,75(2s, 1,5 et 1,5H) ; 0,83(d,3H,J=7Hz) ; 0,97(d,3H,J=7Hz) ; 0,97(s,3H) ; 1,75(s,3H) ; 2,14(d,1H,J=11Hz) ; 3,55(m,2H) ; 4,26(dxd,1H,$J_1$=7Hz,$J_2$=4Hz) ; 4,91(s,1H) ; 5,05(s,1H) delta ppm ;
SM : $M^+$ = 240(0) ; m/e : 139(18), 83(100), 69(50), 55(28), 41(14).

Qualification olfactive : boisé.

19) 3-méthyl-1-(2',2',c-3',t-6'-tétraméthyl-r-1'-cyclohexyloxy)-4-pentén-2-ol

à partir de chlorure de crotyle et de 10,0 g (50 mmole) de l'aldéhyde préparé selon le procédé indiqué ci-après, dans le THF (11,2 g). Après distillation sur résidu au four à boules, puis finement à la colonne à bande tournante, on a isolé 6,9 g d'un mélange contenant 96% de l'alcool désiré, 2% de 3-méthyl-1-(2',2',t-3',t-6'-tétraméthyl-r-1'-cyclohexyloxy)-4-pentén-2-ol et 2% de 3-méthyl-1-(2',2',c-3',c-6'-tétraméthyl-r-1'-cyclohexyloxy)-4-pentén-2-ol.
Rend. 49% ; P. éb. 64°/2 Pa.
IR : 3500, 3100, 2920, 1640, 1100 $cm^{-1}$ ;

Isomère c-3',t-6' (2 diastéréoisomères -OH, thréo/érythro 52%/44%)

| | |
|---|---|
| RMN (360MHz,$^1$H) : | 0,73 et 0,74(2s, 1,3 et 1,7H) ; 0,82(d,3H,J=7Hz) ; 0,96(s,3H) ; 0,97(d,3H,J=7Hz) ; 1,05 et 1,09(2d, 1,7 et 1,3H,J=7Hz) ; 2,32(m,1H) ; 2,42(d,H,J=11Hz) ; 3,56(m,3H) ; 5,05(m,2H) ; 5,74(m,0,41H) ; 5,85(m,052H) delta ppm ; |
| SM (52%) : | $M^+$ = 254(23) ; m/e : 169(17), 139(93), 123(41), 83(100), 69(72), 55(89). |
| SM (44%) : | $M^+$ = 254(19) ; m/e : 169(17), 139(90), 123(39), 83(100), 69(72), 55(86). |

Isomère t-3',t-6'

| | |
|---|---|
| RMN (360MHz,$^1$H) : | 2,77(d,1H,J=11Hz) delta ppm ; |
| SM : | $M^+$ = 254(20) ; m/e : 169(14), 138(77), 123(30), 96(30), 83(95), 69(59), 55(83), 43(100). |
| Qualification olfactive : | boisé. |

20) 1-(2,2,c-3,t-6-tétraméthyl-r-1-cyclohexyloxy)-5-hexén-2-ol

à partir de 4-bromo-1- butène et 5,0 g (25 mmole) de l'aldéhyde préparé selon le procédé indiqué ci-après, dans le THF (6,1 g). La distillation à la colonne à bande tournante a permis d'obtenir 3,4 g d'un mélange à 94% pur, contenant 98% de l'alcool désiré et 2% de 1-(2,2-t-3,t-6-tétraméthyl-r-1-cyclohexyloxy)-5-hexen-2-ol. Ce mélange a ensuite été encore purifié à 99%.
Rend. : 50% ; P. éb. 53°/2,1 Pa.
IR : 3450, 3100, 2920, 1640, 1450, 1100 $cm^{-1}$ ;

Isomère majoritaire (98%)

| | |
|---|---|
| RMN (360MHz,$^1$H) : | 0,73 et 0,74(2s, 1,5 et 1,5H) ; 0,82(d,3H,J=7Hz) ; 0,94(d,3H,J=7Hz) ; 0,95 et 0,98(2s, 1,5 et 1,5H) : 2,2(m,2H) ; 2,42(d,1H,J=11Hz) ; 3,5(m,2H) ; 3,8(m,1H) ; 4,97(d,1H,J=11Hz) ; 5,04(d,1H,J=15Hz) ; 5,83(m,1H) delta ppm ; |
| SM : | $M^+$ = 254(1) ; m/e : 138(54), 123(30), 96(48), 83(100), 69(65), 55(78), 43(70). |

Isomère minoritaire (2%)

| | |
|---|---|
| RMN (360MHz,$^1$H) : | 2,78(d,1H,J=11Hz) delta ppm ; |
| SM : | $M^+$ = 254(0) ; m/e : 138(50), 123(33), 96(33), 83(100), 69(61), 55(90), 43(68). |
| Qualification olfactive : | boisé. |

21) 2-éthyl-1-(2,2,c-3,t-6-tétraméthyl-r-1-cyclohexyloxy)-2-butanol

a) Ce composé a été préparé à partir de la 1-(2,2,c-3,t-6-tétraméthyl-r-1-cyclohexyloxy)-2-butanone [voir préparation sous b), 7,0 g 31 mmole] et de bromoéthane, dans le THF (8,4 g). On a distillé finement à la colonne à bande tournante et obtenu 2,3 g de produit à 95% pur, sous forme d'un mélange contenant 60% du butanol désiré et 40% d'un produit secondaire sans intérêt, le 2-hydroxy-2-méthylbutanoate de 2,2,c-3,t-6-tétraméthyl-r-1-cyclohexyle.
Rend. 28% ; P. éb. 40°/2,5 Pa.
Données analytiques du 2-éthyl-1-(2,2,c-3,t-6-tétraméthyl-r-1-cyclohexy-loxy)-2-butanol :
IR : 3500, 2950, 1460, 1100 $cm^{-1}$ ;
RMN (360MHz,$^1$H) : 0,74(s,3H) ; 0,82(d,3H,J=7Hz) ; 0,87 et 0,88(2t, 3 et 3H,J=7Hz) ;

0,96(d,3H,J=7Hz) ; 0,97(s,3H) ; 2,42(d,1H,J=11Hz) ; 3,43(AB,2H, $J_1$=7Hz, $J_2$=15Hz) delta ppm ;
SM : $M^+$ = 256(0) ; m/e : 170(26), 139(29), 87(85), 83(100), 69(43), 55(43), 43(28).

b) 1-(2,2,c-3,t-6-tétraméthyl-r-1-cyclohexyloxy)-2-butanone Dans un ballon à 4 cols, muni d'une agitation mécanique, on a chargé 900 ml d'acide acétique concentré et 100 ml d'eau. On a ajouté 97 g (0,97 mole) de trioxyde de chrome et refroidi à 0° (mélange glace et sel). On a introduit goutte à goutte 55,25 g (0,24 mole) de 1-(2,2,c-3,t-6-tétraméthyl-r-1-cyclohexyloxy)-2-butanol [voir 3)] sans dépasser 0°. On a continué l'agitation pendant la nuit, à température ambiante. Ensuite, on a ajouté du NaOH à 30%, sans dépasser 20°, pour marquer alcalin, extrait à l'éther, lavé à l'eau à neutralité, séché et concentré. On a ainsi obtenu 44,0 g de produit brut qui a été purifié par distillation sur résidu sur une colonne Vigreux pour fournir 26,45 g d'un mélange à 76% pur contenant 70% de la butanone désirée et 30% d'un produit secondaire, le 2-oxobutanoate de 2,2,c-3,t-6-tétraméthyl-r-1-cyclohexyle.

Rend. 37% ; P. éb. 64°/6,6 Pa.

Données analytiques de la 1-(2,2,c-3,t-6-tétraméthyl-r-1-cyclohexyloxy)-2-butanone :

RMN (360MHz,$^1$H) : 0,8(s,3H) ; 0,83(d,3H,J=7Hz) ; 0,93(d,3H,J=7Hz) ; 0,95(s,3H) ; 1,07(t,3H,J=7Hz) ; 2,42(d,1H,J=11Hz) ; 2,62(q,2H,J=7Hz) ; 4,14(AB,2H,$J_1$=15Hz,$J_2$=26Hz) delta ppm ;
SM : $M^+$ = 226(1) ; m/e : 155(9), 139(28), 83(100), 69(33), 57(30), 43(14).

22) 3-(2,2,c-3,t-6-tétraméthyl-r-1-cyclohexyloxyméthyl)-1-pentén-3-ol

Préparé à partir de bromure de vinyle et 7,0 g (31 mmole) de 1-(2,2,c-3,t-6-tétraméthyl-r-1-cyclohexyloxy)-2-butanone, selon la méthode générale Grignard, dans le THF (8,4 g). On a distillé finement à la colonne à bande tournante et isolé ainsi 2,3 g d'un mélange à 96% pur, contenant 60% du penténol désiré et 40% d'un produit secondaire, le 2-éthyl-2-hydroxy-3-buténoate de 2,2,c-3,t-6-tétraméthyl-r-1-cyclohexyle.

Rend. 28% ; P. éb. 40°/2,3 Pa ;

Données analytiques du 3-(2,2,c-3,t-6-tétraméthyl-r-1-cyclohexyloxyméthyl)-1-pentén-3-ol :

IR : 3500, 2950, 1725, 1460, 1380, 1240, 1100 $cm^{-1}$ ;
RMN (360MHz,$^1$H) ; 0,71 et 0,74(2s, 1,2 et 1,8H) ; 0,81 et 0,82(2d, 1,2 et 1,8H,J=7Hz) ; 0,89(t,3H,J=7Hz) ; 0,95 et 0,97(2s, 1,2 et 1,8H) ; 0,96(d,3H,J=7Hz) ; 2,41 et 2,42(2d, 0,4 et 0,6H,J=11Hz) ; 3,48(AB,2H, $J_1$=7Hz, $J_2$=15Hz) ; 5,17(d,1H,J=11Hz) ; 5,31(d,1H,J=15Hz) ; 5,81(m,1H) delta ppm ;

SM : $M^+$ = 254(0) ; m/e : 139(30), 116(26), 83(100), 69(48), 57(51), 43(34).

Les produits de départ utilisés dans les procédés définis à l'aide des exemples ci-dessus ont été préparés ainsi :

I. Préparation de (2,2,c-3,t-6-tétraméthyl-r-1-cyclohexyloxy)acétaldéhyde

a) 2,2,c-3,t-6-tétraméthyl-r-1-cyclohexanol

A) Dans un ballon muni d'une agitation mécanique, on a chargé 1500 ml de toluène absolu et 92,0 g (4 mole) de sodium coupés en petits dés. On a chauffé à reflux et introduit goutte à goutte le mélange de 154,0 g (1 mole) de 2,2,3,6-tétraméthyl-1-cyclohexanone (80% trans) et de 610 ml (8 mole) d'isopropanol. On a agité 1 h 1/2 à reflux et laissé la nuit à température ambiante. Environ 200 ml d'éthanol ont été ajoutés pour détruire les restes de sodium. Ensuite, on a jeté sur glace, extrait à l'éther, lavé 2 fois à l'eau, concentré sur une colonne Vigreux à pression atmosphérique, puis distillé sous vide humide. On a isolé 151.65 g d'un mélange d'alcools à 96% pur, contenant 73% de l'isomère désiré et 27% d'autres isomères.

Rend. 93% ; P. éb. 64°/6,8 x $10^2$ Pa.

B) Dans un ballon muni d'une agitation mécanique, on a chargé 7,63 g (1,09 mole) de lithium et condensé 1200 ml d'ammoniac à travers un réfrigérant à $CO_2$/acétone. On a introduit goutte à goutte le mélange de 60,0 g (0,39 mole) de 2,2,3,6-tétraméthylcyclohexanone (80% trans), de 45 ml (0,59 mole) d'isopropanol et 80 ml d'éther en laissant refluer l'ammoniac. On a laissé évaporer l'ammoniac durant le week-end, ajouté un peu d'isopropanol pour détruire l'excès de lithium, jeté sur glace, extrait à l'éther, lavé avec $NH_4Cl$ sat. à neutralité, séché, concentré en distillant sur une colonne Vigreux 25 cm. On a isolé ainsi 60,5 g d'un mélange d'alcools 96% pur sous forme cristalline, contenant environ 79% de 2,2,c-3,t-6-tétraméthyl-r-1-cyclohexanol, 17% de 2,2,t-3,t-6-tétraméthyl-r-1-cyclohexanol, 3% de 2,2,c-3,c-6-tétraméthyl-r-1-cyclohexanol et 1% de 2,2,t-3,c-6-tétraméthyl-r-1-cyclohexanol (rend. 95% ; P. éb. 73°/6,2 x $10^2$ Pa).

La recristallisation de ce mélange dans le pentane, deux fois, a permis d'obtenir l'isomère désiré pur avec 58% de rendement.

Les données analytiques du 2,2,c-3,t-6-tétraméthyl-r-1-cyclohexanol étaient les suivants :

| | |
|---|---|
| P. f. : | 57-58° |
| IR : | 3300 (-OH) $cm^{-1}$ |
| RMN (360 MHz,$^1$H) : | 0,73(s,3H) ; 0,85(d,3H,J=6,5Hz) ; 0,97(d,3H,J=6,5Hz) ; 0,99(s,3H) ; 2,76(d,1H,J=environ 10,4Hz) delta ppm ; |
| SM : | $M^+$ = 156(30) ; m/e : 138(9), 123(41), 109(15), 99(5), 95(28), 91(1), 87(2), 83(31), 77(3), 70(52), 67(13), 65(2), 57(51), 55(76), 53(13), 51(2), 45(15), 43(93), 41(100), 39(47). |

b) r-1-allyloxy-2,2,c-3,t-6-tétraméthylcyclohexane

C) Dans un ballon muni d'une agitation mécanique, on a chargé 60,0 g (0,38 mole) du mélange obtenu en a) B) et 600 ml de THF absolu. On a introduit goutte à goutte 282 ml (0,42 mole) de butyllithium 1,50 M dans l'hexane (la température est montée à 45°). Après avoir agité 1 h à température ambiante, on a chauffé à reflux, introduit goutte à goutte 36 ml (0,42 mole) de bromure d'allyle et agité une nuit à reflux. On a ajouté ensuite 22 ml de bromure d'allyle (+60%) goutte à goutte à reflux. L'évolution de la réaction a été suivie par C.G. Après 6 h, on a ajouté à nouveau 20 ml de bromure (+55%) et agité tout le week-end à reflux. On a refroidi à température ambiante, jeté sur glace, extrait à l'éther, lavé à l'eau à neutralité, séché et concentré. On a obtenu 88,9 g de produit brut qui a été distillé sur une colonne de 20 cm remplie d'hélices. On a isolé 50,45 g d'allyloxy 93% pur, comme un mélange 88:12 de r-1-allyloxy-2,2,c-3,t-6- tétraméthylcyclohexane et r-1-allyloxy-2,2,t-3,t-6-tétraméthylcyclohexane, respectivement.

Rend. 62% ; P. éb. 73°/6,2 x $10^2$ Pa

| | |
|---|---|
| IR : | 1090 (-O-) $cm^{-1}$ |
| RMN (360 MHz,$^1$H) : | 0,76(s,3H) ; 0,82(d,3H,J=6Hz) ; 0,96(d,3H,J=6Hz) ; 0,97 (s,3H) ; 2,43(d,0,88H,J=environ 10,8Hz) ; 2,78(d,0,12H,J=environ 10,8Hz) ; 4,08(m,2H) ; 5,13(d,1H,J=9Hz) ; 5,28(large d,1H,J=16Hz) ; 5,96(m,1H) delta ppm ; |
| SM : | $M^+$ = 196(10) ; m/e : 138(14), 123(14), 111(17), 96(16), 83(20), 69(45), 50(52), 41(100). |

D) Dans un ballon à 4 cols, muni d'une agitation mécanique, on a chargé 360 ml de THF, 720 ml de toluène (solution 1:2), 315,75 g (2,02 mole) du mélange d'alcools obtenu selon a) B) et 15,6 g (2,2 mole) de lithium granulés. On a chauffé à 35° et introduit goutte à goutte une solution de 231,6 g de styrène dans 360 ml de THF, lentement à l'aide d'une pompe "CFG ProMinent" à 140 ml/h. On a chauffé 1 h 1/2 à reflux jusqu'à disparition du lithium et introduit lentement à reflux 240 ml (2,8 mole) de bromure d'allyle dans 230 ml de DMSO (diméthylsulfoxyde) à 100 ml/h. On a chauffé encore 1 h à reflux, refroidi, versé sur glace, extrait à l'éther, lavé à l'eau à neutralité, séché et concentré. On a obtenu 533,1 g de produit brut qui a été distillé sur une colonne remplie d'hélices de 20 cm, munie d'une tête à reflux total. On a isolé ainsi 368,95 g de produit 92% pur (rend. 85% ; P. éb. 77°/4,4 x $10^2$ Pa). Les données analytiques étaient identiques à celles présentées dans la section précédente.

c) Dans un ballon à double paroi muni d'une agitation mécanique, on a chargé 79,65 g (0,406 mole) du produit obtenu selon b) (93% pur), 700 ml de méthanol et refroidi à -10°. On a fait barboter un courant d'ozone pendant 3 h. On a purgé à l'azote et versé petit à petit le mélange réactionnel sur une solution de 56,3 g (0,45 mole) de sulfite de sodium dans 415 ml d'eau à 0°. Après avoir concentré le méthanol, on a repris dans un décanteur, lavé 1 fois à l'eau, séché, concentré et on a obtenu 87,4 g de produit brut qui a été utilisé directement dans les réactions de Grignard. Quelques grammes de produit pur ont été isolés, comme un mélange 80:20 de (2,2,c-3,t-6-tétraméthyl-r-1-cyclohexyloxy)acétaldéhyde et (2,2,t-3,t-6-tétraméthyl-r-1-cyclohexyloxy) acétaldéhyde, respectivement.

| | |
|---|---|
| IR : | 1720 (-CHO), 1100 (-O-) $cm^{-1}$ |
| RMN(360MHz,$^1$H) : | 0,74-0,98(m,12H) ; 2,47(d, 0,8H,J=environ 10,8Hz), 2,81(d, 0,2H,J=environ 10,8Hz) ; 4,18(AB,2H,$J_1$=18Hz,$\Delta\nu$ =29Hz,) ; 9,78(s,1H) delta ppm ; |
| SM : | $M^+$= 198(9) ; m/e : 169(2), 154(3), 139(62), 123(18), 113(18), 109(9), 97(17), 83(75), 69(51), 55(98), 41(100). |

II. Préparation de 1-(2,2,c-3,t-6-tétraméthyl-r-1-cyclohexyloxy)-2-propanone

a) r-1,2,2,t-4-tétraméthyl-c-3-(2-méthyl-2-propényloxy)cyclohexane

Dans un ballon à 4 cols muni d'une agitation mécanique, on a chargé 12,5 ml de THF, 25 ml de toluène, 10,0 g (64 mmole) de l'alcool préparé selon l'exemple I.a) ci-dessus et 0,5 g (70 mmole) de lithium granulé.

On a chauffé à 35° et introduit goutte à goutte une solution de 7,3 g (70 mmole) de styrène dans 12,5 ml de THF à l'aide d'un "Perfusor IV" à 12 ml/h, sans dépasser 35° (la température est redescendue au cours de l'introduction). Le mélange de la réaction a été chauffé 30 min à reflux jusqu'à disparition du lithium et on a introduit goutte à goutte une solution de 8,8 ml (90 mmole) de chlorure de méthallyle dans 7,5 ml de DMSO à reflux. On a chauffé encore 4 h à reflux, refroidi, versé sur glace, extrait à l'éther, lavé à l'eau à neutralité, séché et concentré. 32,3 g de produit brut ont été obtenus. Après distillation sur une colonne de 15 cm remplie d'hélices, on a isolé 9,6 g de produit 78% pur, sous forme d'un mélange 79:21 de r-1,2,2,t-4-tétraméthyl-c-3-(2-méthyl-2-propényloxy)cyclohexane et r-1,2,2,c-4-tétraméthyl-t-3-(2-méthyl-2-propényloxy)cyclohexane, respectivement. Une purification supplémentaire a permis d'obtenir ce mélange avec une pureté de 97%.
Rend. 56% ; P. éb. 98°/17 x $10^2$ Pa.

| | |
|---|---|
| IR : | 3150, 2990, 1660, 1455, 1110, 900 $cm^{-1}$ ; |
| RMN(360 MHz,$^1$H) : | isomère majoritaire :<br>0,77(s,3H) ; 0,83(d,3H,J=7Hz) ; 0,96(d,3H,J=7Hz) ; 0,97(s,3H) ; 1,77(s,3H) ; 2,41(d,1H,J=11Hz) ; 3,97(AB,2H,$J_1$=11Hz,$J_2$=40Hz) ; 4,84(s,1H) ; 5,02(s,1H) delta ppm ;<br>isomère minoritaire : 0,97(s,3H) ; 2,77(d,1H,J=11Hz) ; 3,83(AB,2H,$J_1$=11Hz,$J_2$=25Hz) ; 4,84(s,1H) ; 5,00(s,1H) delta ppm ; |
| SM (isomère majoritaire) : | $M^+$ = 210(8) ; m/e : 138(22), 96(25), 83(43), 69(48), 55(100), 43(52) ; |
| SM (isomère minoritaire) : | $M^+$ = 210(8) ; m/e : 138(24), 96(23), 83(39), 69(47), 55(100), 43(52). |

b) Dans un ballon muni d'une agitation mécanique, on a fait barboter un courant d'ozone pendant 1 h, à -10°, dans une solution de 19,2 g (91 mmole) du composé préparé selon a) et 190 ml de méthanol. Pendant ce temps, dans un autre ballon, on a préparé une solution de 11,3 g (0,1 mole) de sulfite de sodium dans 90 ml d'eau. Cette solution a été refroidie à 0° (bain d'eau/glace). On a introduit goutte à goutte l'ozonide formé dans le premier ballon, sans que la température dépasse 20°. On a extrait à l'éther, lavé à l'eau, séché et concentré pour obtenir 41,7 g de produit brut. On a distillé sur résidu et identifié par chromatographie gazeuse. On a isolé 16,4 g de produit à 83% pur, sous forme d'un mélange contenant 68% de 1-(2,2,c-3,t-6-tétraméthyl-r-1-cyclohexyloxy)-2-propanone, 20% de 1-(2,2,t-3,t-6-tétraméthyl-r-1-cyclohexyloxy)-2-propanone, 9% de 1-(2,2,t-3,c-6-tétraméthyl-r-1-cyclohexyloxy)-2-propanone et 3% de 1-(2,2,c-3,c-6-tétraméthyl-r-1-cyclohexyloxy)-2-propanone. Une nouvelle purification de ce produit a permis de porter sa pureté à 96%.
Rend. 70% ; P. éb. 56°/5,4 Pa.
IR : 2940, 1720, 1450, 1350, 1100 $cm^{-1}$

Isomère c-3,t-6 (68%) :

RMN(360MHz,$^1$H) : 0,8(s,3H) ; 0,84(d,3H,J=7Hz) ; 0,94(d,3H,J=7Hz) ; 0,95(s,3H) ; 2,23(s,3H) ; 2,43(d,1H,J=11Hz) ; 4,14(AB,2H,$J_1$=18Hz, $J_2$=25Hz) delta ppm ;
SM : $M^+$ = 212(3) ; m/e : 155(10), 139(49), 83(100), 69(52), 55(49), 43(37).

Isomère t-3,t-6 (20%) :

RMN(360MHz, $^1$H) : 0,94(s,3H) ; 2,25(s,3H) ; 2,78(d,1H,J=11Hz) ; 4,08(AB,2H,$J_1$=15Hz,$J_2$=8Hz) delta ppm ;
SM : $M^+$ = 212(3) ; m/e : 155(11), 139(50), 83(100), 69(65), 55(53), 43(40).

Isomère t-3,c-6 (9%) :

SM : $M^+$ = 212(2) ; m/e : 155(26), 139(30), 83(100), 69(56), 55(52), 43(38).

Isomère c-3,c-6 (3%) :

SM : $M^+$ = 212(2) ; m/e : 155(8), 139(40), 83(100), 69(52), 55(48), 43(39).

c) Par oxydation de 1-(2,2,c-3,t-6-tétraméthyl-r-1-cyclohexyloxy)-2-propanol

La propanone désirée a également été préparée par oxydation de l'alcool susmentionné, dont la préparation est décrite à l'Exemple 13, selon la méthode suivante : dans un ballon à 3 cols muni d'une agitation magnétique, on a chargé 9,1 g (42 mole) de PCC (chlorochromate de pyridinium) et 10 ml de chlorure de méthylène. On a introduit goutte à goutte 6,0 g (28 mmole) de l'alcool susmentionné dans 60 ml de chlorure de méthylène. La température est montée à 25°. On a laissé le mélange réactionnel sous agitation pendant le week-end à température ambiante, ensuite filtré sur colonne de $SiO_2$, rincé à l'éther et concentré. On a obtenu 6,0 g de produit brut, qui ont fourni après distillation sur colonne Vigreux 3,6 g de produit 86% pur.
Rend. 52% ; P. éb. 51°/5,6 x $10^2$ Pa.

Exemple 23

Préparation d'une composition parfumante

On a préparé une composition parfumante de base de type fleuri à partir des ingrédients suivants :

| Ingrédients | Parties en poids |
|---|---|
| Salicylate de benzyle | 120 |
| Exaltex (marque enregistrée) 1) | 100 |
| Iralia (marque enregistrée) 2) | 100 |
| Alcool phényléthylique | 100 |
| Mayol (marque enregistrée) 3) | 70 |
| Hedione (marque enregistrée) 4) | 60 |
| Acétate de p-tert-butyl-cyclohexyle | 50 |
| Acétate de benzyle | 40 |
| Acétate de terpényle | 30 |
| Acétate de carbinol BDM | 30 |
| Essence de girofle Madagascar distillée | 25 |
| Héliotropine | 25 |
| Citronellol | 25 |
| Acétate de géranyle | 20 |
| Alcool cinnamique | 20 |
| Nonaldéhyde à 10% * | 20 |
| Aldéhyde anisique à 10% * | 15 |
| Coumarine | 15 |
| Ethylvanilline à 1% * | 10 |
| Isobutylquinoleïne à 1% * | 10 |
| Rosinol cristallin | 10 |
| Damascone bêta à 10% * | 5 |
| Total | 900 |

* dans le dipropylène glycol (DIPG)

1) pentadécanolide ; origine : Firmenich SA, Genève, Suisse

2) méthylionone ; origine : Firmenich SA, Genève, Suisse

3) hydroxyméthyle isopropyle cyclohexane ; origine : Firmenich SA ; Genève, Suisse

4) dihydrojasmonate de méthyle ; origine : Firmenich SA, Genève, Suisse

A partir de cette composition de base, on a préparé 8 compositions parfumantes, avec deux ingrédients connus de l'art antérieur, à savoir le 1-(2,2,c-3,t-6-tétraméthyl-r-1-cyclohexyl)-3-hexanol et le 1-(2,2,3,6-tétraméthyl-cyclohexyloxy)-2-pentanol [mélange contenant environ 51% de l'isomère (c-3,t-6), 28% de (t-3,c-6) et 21% de (c-3,c-6)]et six composés préférés selon la présente invention. Ces compositions sont décrites dans le Tableau II suivant, dans lequel les composés selon l'invention sont désignés par leur chiffre de référence cité au Tableau I.

Tableau II

| Ingrédients | Compositions | | | | | | | |
|---|---|---|---|---|---|---|---|---|
| | A | B | C | D | E | F | G | H |
| Base fleurie | 900 | 900 | 900 | 900 | 900 | 900 | 900 | 900 |
| 1-(2,2,c-3,t-6-tétraméthyl-r-1-cyclohexyl)-3-hexanol à 1% * | 10 | - | - | - | - | - | - | - |
| 1-(2,2,3,6-tétraméthyl-cyclohexyloxy)-2-pentanol à 10% * | - | 10 | - | - | - | - | - | - |
| 1) à 10% * | - | - | 10 | - | - | - | - | - |
| 2) à 10% * | - | - | - | 10 | - | - | - | - |
| 3) à 10% * | - | - | - | - | 10 | - | - | - |
| 5) à 10% * | - | - | - | - | - | 10 | - | - |
| 6) à 10% * | - | - | - | - | - | - | 10 | - |
| 8) à 10% * | - | - | - | - | - | - | - | 10 |
| Total | 910 | 910 | 910 | 910 | 910 | 910 | 910 | 910 |

* dans le DIPG

Lorsqu'on ajoute les composés selon l'invention à la composition de base possédant une note odorante fleurie, cette dernière développe alors une note nouvelle, plus riche et boisée, devenant ainsi plus intéressante et arrondie. Cet effet a été remarqué dans toutes les compositions C à H, par rapport à la composition de base.

Lorsque les experts parfumeurs ont comparé entre elles des compositions C à H, ils ont montré une préférence pour les trois premières, à savoir C, D et E, avec une préférence toute particulière pour la composition C. Celle-ci développait, par ailleurs, une note bien supérieure à celle de la composition B, une note plus nette et plus élégante. Ce même effet a été remarqué lorsqu'on a comparé les compositions D à H avec la composition B. Par rapport à la composition A, presque toutes les compositions C à H ont été jugées d'un emploi plus facile, en dépit du fait que l'ingrédient parfumant de la composition A était dix fois plus dilué que les autres ingrédients parfumants, afin de compenser l'effet de la puissance remarquable de sa note odorante. Les compositions C à E, en particulier, ont été jugées très intéressantes pour un emploi alternatif à celui de la composition A, leur odeur s'avérant moins agressive, plus fine et discrète que celle de cette dernière, tout en gardant la tonalité chaude et riche du fond boisé-ambré.

Exemple 24

Préparation d'un savon

A un savon en copeaux, obtenu à partir d'une base de savon au sodium préparée avec de l'huile de coco et de suif, on a ajouté environ 0,1 à 0,5%, en poids, par rapport au poids de savon et suivant l'ingrédient parfumant utilisé, de l'un des composés selon l'invention. La note originale du savon s'est trouvée alors couverte par une note agréablement parfumée, à odeur boisée-ambrée fleurie.

## Revendications

**1.** Composé de formule

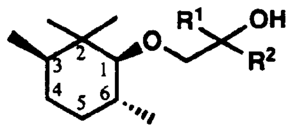

(I)

dans laquelle

$R^1$ représente un atome d'hydrogène ou un radical alkyle saturé de $C_1$ à $C_4$, linéaire ou ramifié, et $R^2$ représente un radical alkyle inférieur, saturé ou insaturé, linéaire ou ramifié,

ou tout mélange contenant une proportion prépondérante dudit composé accompagné de son isomère de formule

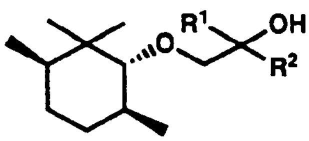

(II)

dans laquelle $R^1$ et $R^2$ sont définis comme à la formule (I).

**2.** Mélange selon la revendication 1, caractérisé en ce qu'il contient, à raison de 75% ou plus en poids, un composé de formule (I) définie à la revendication 1, accompagné d'une quantité inférieure ou égale à 25% en poids de l'isomère de formule (II) définie à la revendication 1.

**3.** Mélange selon la revendication 1, caractérisé en ce qu'il contient à titre additionnel un composé de formule

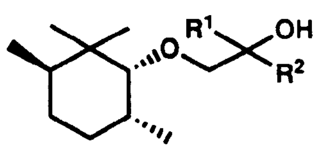

et/ou

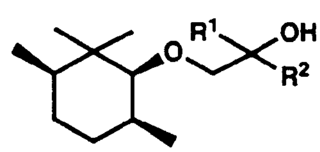

(III) (IV)

dans lesquelles $R^1$ et $R^2$ sont définis comme à la formule (I), dans une proportion inférieure ou égale à 10% en poids.

**4.** A titre de composé selon la revendication 1, l'un des composés suivants :

a. 1-(2,2,c-3,t-6-tétraméthyl-r-1-cyclohexyloxy)-2-pentanol ;
b. 1-(2,2,c-3,t-6-tétraméthyl-r-1-cyclohexyloxy)-4-pentén-2-ol ;
c. 1-(2,2,c-3,t-6-tétraméthyl-r-1-cyclohexyloxy)-2-butanol

ou un mélange contenant une quantité prépondérante dudit composé.

**5.** Utilisation d'un composé de formule (I) ou d'un mélange selon la revendication 1 à titre d'ingrédient parfumant.

**6.** Composition parfumante contenant à titre d'ingrédient parfumant un composé de formule (I) ou un mélange selon la revendication 1.

**7.** Produit parfumé contenant à titre d'ingrédient parfumant un composé de formule (I) ou un mélange selon la revendication 1.

**8.** A titre de produit parfumé selon la revendication 7, un parfum, une eau de toilette, un savon, un gel de

douche au bain, un shampoing, un désodorisant corporel ou d'air ambiant, une préparation cosmétique, un détergent ou un adoucissant textile.

9. Procédé de préparation d'un composé de formule (I) ou d'un mélange selon la revendication 1, caractérisé en ce qu'on fait réagir, dans les conditions d'une réaction de Grignard, un composé de formule

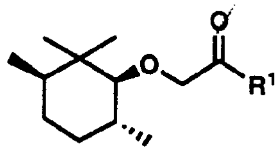

(VI)

dans laquelle le symbole $R^1$ est défini comme à la formule (I), ou un mélange contenant des quantités prépondérantes en ledit composé (VI), avec un composé organométallique de formule $R^2MgX$, dans laquelle X représente un atome d'halogène et $R^2$ est défini comme à la formule (I).

10. Composé de formule (VI) telle que définie à la revendication 9 ou mélange riche en ce composé.

## Claims

1. Compound of formula

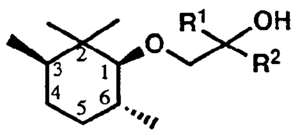

(I)

wherein

$R^1$ represents a hydrogen atom or a $C_1$ to $C_4$ saturated, linear or branched, alkyl radical and $R^2$ designates a lower alkyl radical, saturated or unsaturated, linear or branched;

or any mixture containing a predominant amount of said compound together with its isomer of formula

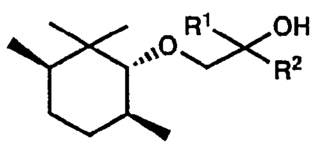

(II)

wherein $R^1$ and $R^2$ are defined as in formula (I).

2. A mixture according to claim 1, characterized in that it contains 75% or more, by weight, of a compound of formula (I) as defined in claim 1, together with about 25% or less, by weight, of its isomer of formula (II) as defined in claim 1.

3. A mixture according to claim 1, characterized in that it further contains a compound of formula

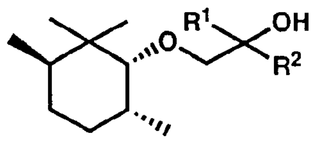

and/or

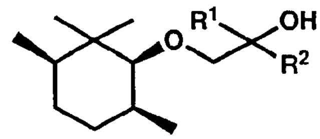

(III) (IV)

wherein $R^1$ and $R^2$ are defined as in formula (I), in an amount lower than or equal to 10% by weight.

4. As a compound according to claim 1, one of the following compounds:
   a. 1-(2,2,c-3,t-6-tetramethyl-r-1-cyclohexyloxy)-2-pentanol ;
   b. 1-(2,2,c-3,t-6-tetramethyl-r-1-cyclohexyloxy)-4-penten-2-ol ;
   c. 1 -(2,2,c-3,t-6-tetramethyl-r-1-cyclohexyloxy)-2-butanol or a mixture containing a predominant amount of said compound.

5. Use of a compound of formula (I), or of a mixture according to claim 1 as a perfuming ingredient.

6. Perfuming composition containing as perfuming ingredient a compound of formula (I) or a mixture according to claim 1.

7. Perfumed article containing as perfuming ingredient a compound of formula (I) or a mixture according to claim 1.

8. As a perfumed article according to claim 7, a perfume or Cologne, a soap, a shower or bath gel, a shampoo, a body or air deodorant, a cosmetic preparation, a detergent or a fabric softener.

9. Process for the preparation of a compound of formula (I) or of a mixture according to claim 1, characterized in that a compound of formula

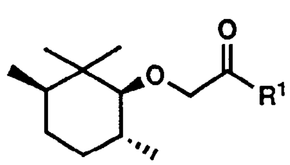

(VI)

wherein symbol $R^1$ is defined as in formula (I),
or a mixture containing a predominant amount of said compound (VI), is reacted, under the conditions of a Grignard reaction, with an organometallic compound of formula $R^2MgX$, wherein X stands for a halogen atom and $R^2$ is defined as in formula (I).

10. A compound of formula (VI) as defined in claim 9, or any mixture rich in this compound.

## Patentansprüche

1. Verbindung der Formel

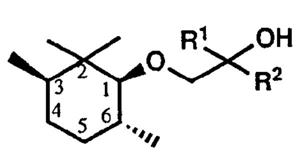

(I),

worin

$R^1$ ein Wasserstoffatom oder einen $C_1$ bis $C_4$ linearen oder verzweigten gesättigten Alkylrest bedeutet, und $R^2$ einen niedrigen, gesättigten oder ungesättigten, linearen oder verzweigten Alkylrest bedeutet,
oder jede Mischung, die eine überwiegende Menge der obengenannten Verbindung mit ihrem Isomeren der Formel

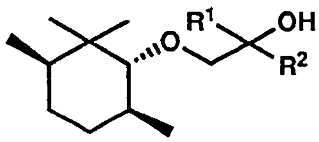

(II) ,

worin $R^1$ und $R^2$ wie in der Formel (I) definiert sind, enthält.

**2.** Mischung gemäß Anspruch 1, dadurch gekennzeichnet, daß sie eine Verbindung der Formel (I) nach Anspruch 1 in einer Menge von 75 Gewichtsprozenten oder mehr, begleitet von dem Isomeren der Formel (II) nach Anspruch 1 in einer Menge von 25 Gewichtsprozenten oder weniger enthält.

**3.** Mischung gemäß Anspruch 1, dadurch gekennzeichnet, daß sie zusätzlich eine Verbindung der Formel

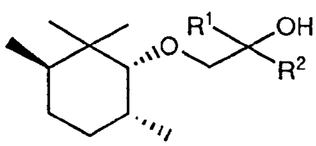

(III)

und/oder

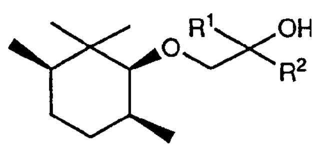

(IV) ,

in welcher $R^1$ und $R^2$ wie in der Formel (I) definiert sind, in einer Proportion von 10 Gewichtsprozenten oder weniger enthält.

**4.** Als Verbindung gemäß Anspruch 1, eine der folgenden Verbindungen:
a. 1-(2,2,c-3,t-6-tetramethyl-r-1-cyclohexyloxy)-2-pentanol ;
b. 1-(2,2,c-3,t-6-tetramethyl-r-1-cyclohexyloxy)-4-penten-2-ol ;
c. 1-(2,2,c-3,t-6-tetramethyl-r-1-cyclohexyloxy)-2-butanol
oder eine Mischung, die eine überwiegende Menge der obengenannten Verbindung enthält.

**5.** Verwendung einer Verbindung der Formel (I) oder einer Mischung gemäß Anspruch 1 als Riechstoff.

**6.** Riechstoffkomposition, die als Riechstoff eine Verbindung der Formel (I) oder eine Mischung gemäß Anspruch 1 enthält.

**7.** Parfümiertes Produkt, welches als Riechstoff eine Verbindung der Formel (I) oder eine Mischung gemäß Anspruch 1 enthält.

**8.** Als parfümiertes Produkt gemäß Anspruch 7, ein Parfüm oder ein Kölnischwasser, eine Seife, ein Bad- oder Duschgel, ein Shampoo, einen Körper- oder Raumbedufter, ein kosmetisches Präparat, ein Reinigungsmittel oder ein Textilweichmacher.

**9.** Verfahren zur Herstellung einer Verbindung der Formel (I) oder einer Mischung gemäß Anspruch 1, dadurch gekennzeichnet, daß man eine Verbindung der Formel

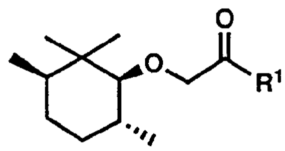

(VI),

worin das Symbol $R^1$ wie in der Formel (I) definiert ist, oder eine Mischung, die eine überwiegende Menge der Verbindung (VI) enthält, mit einer metallorganischen Verbindung der Formel $R^2MgX$, in welcher X ein Halogenatom bedeutet und $R^2$ wie in der Formel (I) definiert ist, unter Grignard Reaktionsbedigungen, zur Reaktion bringt.

**10.** Verbindung der Formel (VI) wie im Anspruch 9 definiert oder eine an dieser Verbindung reiche Mischung.